# EUROPÄISCHE PATENTANMELDUNG

(11) **EP 1 106 694 A1**
(43) Veröffentlichungstag der Anmeldung: **13.06.2001**
(21) Anmeldenummer: 00125716.1
(22) Anmeldetag: 24.11.2000
(51) Int. Cl.: C12N 15/54, C12N 9/12, C12P 13/04, C12P 13/08

(54) **Für das glk-Gen codierende Nukleotidsequenzen**

(30) Priorität: 02.12.1999 DE 19958159
(71) Anmelder: Degussa AG, 40474 Düsseldorf (DE)
(72) Erfinder: Möckel, Bettina, Dr., 40597 Düsseldorf (DE); Pfefferle, Walter, Dr., 33790 Halle (Westf.) (DE)

(57) **Zusammenfassung**

Die Erfindung betrifft ein isoliertes Polynukleotid, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70 % identisch ist mit der Aminosäuresequenz von SEQ ID No.2,
c) Polynukleotid, das komplementär ist zu den Polynukleotiden von a) oder b), und
d) Polynukleotid, enthaltend mindestens 15 aufeinanderfolgende Basen der Polynukleotidsequenz von a), b) oder c),
und Verfahren zur fermentativen Herstellung von L-Aminosäuren unter Verstärkung des für das Enzym Glukokinase codierenden glk-Gens.

SEQUENZPROTOKOLL

<110> Degussa-Hüls AG

<120> Neue für das glk Gen codierende Nukleotidsequenzen

<130> 990177 BT

<140>
<141>

<160> 2

<170> PatentIn Ver. 2.1

<210> 1
<211> 1540
<212> DNA
<213> Corynebacterium glutamicum

<220>
<221> CDS
<222> (461)..(1429)

<400> 1
   aaattccttg ggcgccttga atatcaagat atgatcaaca cgcttgccgc cgcagatatt 60
   ttcgcgatgc cagcgcgcac ccgcggtggc ggacttgatg ttgaaggctt gggcattgtc 120
   tatctcgagg cacaagcctg cggagtgccg gtgatagccg gcacctctgg cggcgcgcca 180
   gagacggtga ctccggcaac tggcctggtt gtggaggggt cggacgtcga taagctgtct 240
   gagcttttaa ttgagcttct cgacgatccg atccgccgcg ccgcgatggg cgctgcaggt 300
   agggcgcatg tggaggccga atggtcgtgg gaaatcatgg gggagcggtt gaccaatatt 360
   ttgcagagtg aaccacgatg atggttggac agctgttgat agctatactt tgaaagatta 420
   aattcaccta aatcctgtgt agaacgcgag ggcactcttt atg cca caa aaa ccg 475
                                              Met Pro Gln Lys Pro
                                                1               5
   gcc agt ttc gcg gtg ggc ttt gac atc ggc ggc acc aac atg cga gcc 523
   Ala Ser Phe Ala Val Gly Phe Asp Ile Gly Gly Thr Asn Met Arg Ala
                    10                  15                  20
   ggg ctt gtc gac gaa tcc ggg cgc atc gtg acc agt ttg tcg gcg ccg 571
   Gly Leu Val Asp Glu Ser Gly Arg Ile Val Thr Ser Leu Ser Ala Pro
                25                  30                  35
   tcg ccg cgc acg acg cag gca atg gaa cag ggg att ttt gat cta gtc 619
   Ser Pro Arg Thr Thr Gln Ala Met Glu Gln Gly Ile Phe Asp Leu Val
            40                  45                  50
   gaa cag ctc aag gcc gaa tac ccg gtt ggt gct gtg gga ctt gcc gtc 667
   Glu Gln Leu Lys Ala Glu Tyr Pro Val Gly Ala Val Gly Leu Ala Val
        55                  60                  65
   gcg gga ttt ttg gat cct gag tgc gag gtt gtt cga ttt gcc ccg cac    715
   Ala Gly Phe Leu Asp Pro Glu Cys Glu Val Val Arg Phe Ala Pro His
    70                  75                  80                  85
   ctt cct tgg cgc gat gag cca gtg cgt gaa aag ttg gaa aac ctt ttg    763
   Leu Pro Trp Arg Asp Glu Pro Val Arg Glu Lys Leu Glu Asn Leu Leu
                    90                  95                 100
   ggc ctg cct gtt cgt ttg gaa cat gat gcc aac tca gca gcg tgg ggt    811
   Gly Leu Pro Val Arg Leu Glu His Asp Ala Asn Ser Ala Ala Trp Gly
               105                 110                 115
   gag cat cgt ttt ggt gca gct caa ggc gct gac aac tgg gtt ttg ttg    859
   Glu His Arg Phe Gly Ala Ala Gln Gly Ala Asp Asn Trp Val Leu Leu
           120                 125                 130
   gca ctc ggc act gga att ggt gca gcg ctg att gaa aaa ggc gaa att    907
   Ala Leu Gly Thr Gly Ile Gly Ala Ala Leu Ile Glu Lys Gly Glu Ile
       135                 140                 145
   tac cgt ggt gca tat ggc acg gca cca gaa ttt ggt cat ttg cgt gtt    955
   Tyr Arg Gly Ala Tyr Gly Thr Ala Pro Glu Phe Gly His Leu Arg Val
   150                 155                 160                 165
   gtt cgt ggc gga cgc gca tgt gcg tgt ggc aaa gaa ggc tgc ctg gag   1003
   Val Arg Gly Gly Arg Ala Cys Ala Cys Gly Lys Glu Gly Cys Leu Glu
                   170                 175                 180
   cgt tac tgt tcc ggt act gcc ttg gtt tac act gcg cgt gaa ttg gct   1051
   Arg Tyr Cys Ser Gly Thr Ala Leu Val Tyr Thr Ala Arg Glu Leu Ala
               185                 190                 195
   tcg cat ggc tca ttc cgc aac agc ggg ctg ttt gac aag atc aaa gcc   1099
   Ser His Gly Ser Phe Arg Asn Ser Gly Leu Phe Asp Lys Ile Lys Ala
           200                 205                 210
   gat ccg aat tcc atc aat gga aaa acg atc act gcg gca gcg cgc caa   1147
   Asp Pro Asn Ser Ile Asn Gly Lys Thr Ile Thr Ala Ala Ala Arg Gln
       215                 220                 225
   gaa gac cca ctt gct ctc gcc gtt ctg gaa gat ttc agc gag tgg ctg   1195
   Glu Asp Pro Leu Ala Leu Ala Val Leu Glu Asp Phe Ser Glu Trp Leu
   230                 235                 240                 245
   ggc gaa act ttg gcg atc att gct gat gtc ctt gac cca ggc atg atc   1243
   Gly Glu Thr Leu Ala Ile Ile Ala Asp Val Leu Asp Pro Gly Met Ile
                   250                 255                 260
   atc att ggt ggc gga ctg tcc aat gct gcc gac ctt tat ttg gat cgc   1291
   Ile Ile Gly Gly Gly Leu Ser Asn Ala Ala Asp Leu Tyr Leu Asp Arg
               265                 270                 275
   tcg gtc aac cac tat tcc acc cgc atc gtc ggc gca gga tat cgc cct   1339
   Ser Val Asn His Tyr Ser Thr Arg Ile Val Gly Ala Gly Tyr Arg Pro
           280                 285                 290
   ttg gca cgc gtt gcc aca gct cag ttg ggt gcg gat gct ggc atg atc   1387
   Leu Ala Arg Val Ala Thr Ala Gln Leu Gly Ala Asp Ala Gly Met Ile
       295                 300                 305
   ggt gtc gct gat cta gct cga cgc tct gta gtg gaa gcc aac          1429
   Gly Val Ala Asp Leu Ala Arg Arg Ser Val Val Glu Ala Asn
   310                 315                 320
   taggtgtttt tcggtgggct gcgatgacgc atgtccacca aaagagccac cccttaaaga 1489
   aattaaaaag tggttttggt agcttcgcag caaaatacac atcgtgggta a          1540


<210> 2
<211> 323
<212> PRT
<213> Corynebacterium glutamicum

<400> 2
Met Pro Gln Lys Pro Ala Ser Phe Ala Val Gly Phe Asp Ile Gly Gly
  1               5                  10                  15

Thr Asn Met Arg Ala Gly Leu Val Asp Glu Ser Gly Arg Ile Val Thr
             20                  25                  30

Ser Leu Ser Ala Pro Ser Pro Arg Thr Thr Gln Ala Met Glu Gln Gly
         35                  40                  45

Ile Phe Asp Leu Val Glu Gln Leu Lys Ala Glu Tyr Pro Val Gly Ala
     50                  55                  60

Val Gly Leu Ala Val Ala Gly Phe Leu Asp Pro Glu Cys Glu Val Val
 65                  70                  75                  80

Arg Phe Ala Pro His Leu Pro Trp Arg Asp Glu Pro Val Arg Glu Lys
                 85                  90                  95

Leu Glu Asn Leu Leu Gly Leu Pro Val Arg Leu Glu His Asp Ala Asn
            100                 105                 110

Ser Ala Ala Trp Gly Glu His Arg Phe Gly Ala Ala Gln Gly Ala Asp
        115                 120                 125

Asn Trp Val Leu Leu Ala Leu Gly Thr Gly Ile Gly Ala Ala Leu Ile
    130                 135                 140

Glu Lys Gly Glu Ile Tyr Arg Gly Ala Tyr Gly Thr Ala Pro Glu Phe
145                 150                 155                 160

Gly His Leu Arg Val Val Arg Gly Gly Arg Ala Cys Ala Cys Gly Lys
                165                 170                 175

Glu Gly Cys Leu Glu Arg Tyr Cys Ser Gly Thr Ala Leu Val Tyr Thr
            180                 185                 190

Ala Arg Glu Leu Ala Ser His Gly Ser Phe Arg Asn Ser Gly Leu Phe
        195                 200                 205

Asp Lys Ile Lys Ala Asp Pro Asn Ser Ile Asn Gly Lys Thr Ile Thr
    210                 215                 220

Ala Ala Ala Arg Gln Glu Asp Pro Leu Ala Leu Ala Val Leu Glu Asp
225                 230                 235                 240

Phe Ser Glu Trp Leu Gly Glu Thr Leu Ala Ile Ile Ala Asp Val Leu
                245                 250                 255

Asp Pro Gly Met Ile Ile Ile Gly Gly Gly Leu Ser Asn Ala Ala Asp
            260                 265                 270

Leu Tyr Leu Asp Arg Ser Val Asn His Tyr Ser Thr Arg Ile Val Gly
        275                 280                 285

Ala Gly Tyr Arg Pro Leu Ala Arg Val Ala Thr Ala Gln Leu Gly Ala
    290                 295                 300

Asp Ala Gly Met Ile Gly Val Ala Asp Leu Ala Arg Arg Ser Val Val
305                 310                 315                 320

Glu Ala Asn

## Beschreibung

Gegenstand der Erfindung sind für das glk-Gen codierende Nukleotidsequenzen und Verfahren zur fermentativen Herstellung von L-Aminosäuren, insbesondere L-Lysin, unter Verwendung von coryneformen Bakterien, in denen das glk-Gen verstärkt wird.

### Stand der Technik

L-Aminosäuren, insbesondere L-Lysin, finden in der Humanmedizin und in der pharmazeutischen Industrie, insbesondere aber in der Tierernährung Anwendung.

Es ist bekannt, daß L-Aminosäuren durch Fermentation von Stämmen coryneformer Bakterien, insbesondere Corynebacterium glutamicum hergestellt werden. Wegen der großen Bedeutung wird ständig an der Verbesserung der Herstellverfahren gearbeitet. Verfahrensbesserungen können fermentationstechnische Maßnahmen wie z. B. Rührung und Versorgung mit Sauerstoff, oder die Zusammensetzung der Nährmedien wie z. B. die Zuckerkonzentration während der Fermentation, oder die Aufarbeitung zur Produktform durch z. B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z. B. das Lysin-Analogon S-(2-Aminoethyl)-Cystein oder auxotroph für regulatorisch bedeutsame Metabolite sind und L-Aminosäuren wie z. B. L-Lysin produzieren.

Seit einigen Jahren werden ebenfalls Methoden der rekombinanten DNA-Technik zur Stammverbesserung L-Aminosäure produzierender Stämme von Corynebacterium eingesetzt, indem man einzelne Biosyntheseqene für L-Aminosäure amplifiziert und die Auswirkung auf die L-Aminosäure-Produktion untersucht. Übersichtsartikel hierzu findet man unter anderem bei Kinoshita ("Glutamic Acid Bacteria", in: Biology of Industrial Microorganisms, Demain and Solomon (Eds.), Benjamin Cummings, London, UK, 1985, 115-142), Hilliger (BioTec 2, 40-44 (1991)), Eggeling (Amino Acids 6:261-272 (1994)), Jetten und Sinskey (Critical Reviews in Biotechnology 15, 73-103 (1995)) und Sahm et al. (Annuals of the New York Academy of Science 782, 25-39 (1996)).

### Aufgabe der Erfindung

Die Erfinder haben sich zur Aufgabe gestellt, neue Maßnahmen zur verbesserten fermentativen Herstellung von L-Aminosäuren, insbesondere L-Lysin, bereitzustellen.

### Beschreibung der Erfindung

L-Aminosäuren, insbesondere L-Lysin, finden in der Humanmedizin, in der pharmazeutischen Industrie und insbesondere in der Tierernährung Anwendung. Es besteht daher ein allgemeines Interesse daran, neue verbesserte Verfahren zur Herstellung von L-Aminosäuren, insbesondere L-Lysin, bereitzustellen.

Wenn im folgenden L-Lysin oder Lysin erwähnt werden, sind damit nicht nur die Base sondern auch die Salze wie z. B. Lysin-Monohydrochlorid oder Lysin-Sulfat gemeint.

Gegenstand der Erfindung ist ein isoliertes Polynukleotid aus coryneformen Bakterien, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukieotid, das für ein Polypeptid codiert, das eine Aminosäuresequenz enthält, die zu mindestens 70% identisch ist mit der Aminosäuresequenz von SEQ ID No.2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c).

Gegenstand der Erfindung ist ebenfalls ein Polynukleotid, das eine in coryneformen Bakterien replizierbare, bevorzugt rekombinante, DNA ist.

Gegenstand der Erfindung ist ebenfalls ein Polynukleotid, das eine RNA ist.

Gegenstand der Erfindung ist ebenfalls ein Polynukleotid gemäß Anspruch 1, wobei es sich bevorzugt um eine replizierbare DNA handelt, enthaltend:
(i) die Nukleotidsequenz, gezeigt in SEQ ID No.1, oder
(ii) mindestens eine Sequenz, die der Sequenz (i) innerhalb des Bereichs der Degeneration des genetischen Codes entspricht, oder
(iii) mindestens eine Sequenz, die mit der zur Sequenz (i) oder (ii) komplementären Sequenz hybridisiert, und gegebenenfalls
(iv) funktionsneutrale Sinnmutationen in (i).

Weitere Gegenstände sind ein Vektor, enthaltend das Polypeptid gemäß Anspruch 1, und als Wirtszelle dienende coryneforme Bakterien, die den Vektor enthalten.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank, die das vollständige Gen mit der Polynukleotidsequenz entsprechend SEQ ID No. 1 enthält, mit einer Sonde, die die Sequenz des genannten Polynukleotids gemäß SEQ ID No. 1 oder ein Fragment davon enthält und Isolierung der genannten DNA-Sequenz.

Polynukleotidsequenzen gemäß der Erfindung sind geeignet als Hybridisierungs-Sonden für RNA, cDNA und DNA, um cDNA in voller Länge zu isolieren, die für Glukokinase codieren und solche cDNA oder Gene zu isolieren, die eine hohe Ähnlichkeit der Sequenz mit der des Glukokinase-Gens aufweisen.

Polynukleotidsequenzen gemäß der Erfindung sind weiterhin geeignet als Primer zur Herstellung von DNA von Genen, die für Glukokinase codieren, durch die Polymerase-Kettenreaktion (PCR).

Solche als Sonden oder Primer dienende Oligonukleotide enthalten mindestens 30, bevorzugt mindestens 20, ganz besonders bevorzugt mindestens 15 aufeinanderfolgende Basen. Geeignet sind ebenfalls Oligonukleotide mit einer Länge von mindestens 40 oder 50 Nukleotiden.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Unter "Polypeptiden" versteht man Peptide oder Proteine, die zwei oder mehr über Peptidbindungen verbundene Aminosäuren enthalten.

Die Polypeptide gemäß Erfindung schließen ein Polypeptid gemäß SEQ ID No. 2, insbesondere solche mit der biologischen Aktivität der Glukokinase und auch solche ein, die zu wenigstens 70 % identisch sind mit dem Polypeptid gemäß SEQ ID No. 2, bevorzugt zu wenigstens 80% und besonders bevorzugt die zu wenigstens 90 % bis 95 % Identität mit dem Polypeptid gemäß SEQ ID No. 2 und die genannte Aktivität aufweisen.

Die Erfindung betrifft weiterhin ein Verfahren zur fermentativen Herstellung von L-Aminosäuren, insbesondere L-Lysin, unter Verwendung von coryneformen Bakterien, die insbesondere bereits eine L-Aminosäure produzieren, und in denen die für das glk-Gen codierenden Nukleotidsequenzen verstärkt, insbesondere überexprimiert werden.

Der Begriff "Verstärkung" beschreibt in diesem Zusammenhang die Erhöhung der intrazellulären Aktivität eines oder mehrerer Enzyme in einem Mikroorganismus, die durch die entsprechende DNA codiert werden, indem man beispielsweise die Kopienzahl des Gens bzw. der Gene erhöht, einen starken Promotor verwendet oder ein Gen verwendet, das für ein entsprechendes Enzym mit einer hohen Aktivität codiert und gegebenenfalls diese Maßnahmen kombiniert.

Die Mikroorganismen, die Gegenstand der vorliegenden Erfindung sind, können L-Aminosäuren, insbesondere L-Lysin aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind die zum Beispiel bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 und
Brevibacterium divaricatum ATCC14020
und daraus hergestellte L-Lysin produzierende Mutanten bzw. Stämme, wie beispielsweise
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM5715.

Den Erfindern gelang es, das neue, für das Enzym Glukokinase (EC 2.7.1.2) codierende glk-Gen von C. glutamicum zu isolieren.

Zur Isolierung des glk-Gens oder auch anderer Gene von C. glutamicum wird zunächst eine Genbank dieses Mikrorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495-508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde. Börmann et al. (Molecular Microbiology 6(3), 317-326 (1992)) wiederum beschreiben eine Genbank von C. glutamicum ATCC13032 unter Verwendung des Cosmids pHC79 (Hohn und Collins, Gene 11, 291-298 (1980)). Zur Herstellung einer Genbank von C. glutamicum in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E. coli-Stämme, die restriktions- und rekombinationsdefekt sind. Ein Beispiel hierfür ist der Stamm DH5amcr, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige für die Sequenzierung geeignete Vektoren subkloniert und anschließend sequenziert werden, so wie es z. B. bei Sanger et al. (Proceedings of the National Academy of Sciences of the United States of America, 74:5463-5467, 1977) beschrieben ist.

Auf diese Weise wurde die neue für das Gen glk codierende DNA-Sequenz von C. glutamicum erhalten, die als SEQ ID No. 1 Bestandteil der vorliegenden Erfindung ist. Weiterhin wurde aus der vorliegenden DNA-Sequenz mit den oben beschriebenen Methoden die Aminosäuresequenz des entsprechenden Proteins abgeleitet. In SEQ ID No. 2 ist die sich ergebende Aminosäuresequenz des glk-Genproduktes dargestellt.

Codierende DNA-Sequenzen, die sich aus SEQ ID NO. 1 durch die Degeneriertheit des genetischen Codes ergeben, sind ebenfalls Bestandteil der Erfindung. In der Fachwelt sind weiterhin konservative Aminosäureaustausche wie z. B. Austausch von Glycin gegen Alanin oder von Asparaginsäure gegen Glutaminsäure in Proteinen als "Sinnmutationen" (sense mutations) bekannt, die zu keiner grundsätzlichen Veränderung der Aktivität des Proteins führen, d. h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID NO. 2 ergeben, und diese Aminosäuresequenzen codierende DNA-Sequenzen sind ebenfalls Bestandteil der Erfindung.

In gleicher Weise sind DNA-Sequenzen, die mit SEQ ID NO. 1 oder Teilen von SEQ ID NO. 1 hybridisieren Bestandteil der Erfindung. Schließlich sind DNA-Sequenzen Bestandteil der Erfindung, die durch die Polymerase-Kettenreaktion (PCR) unter Verwendung von Primern hergestellt werden, die sich aus SEQ ID NO. 1 ergeben. Derartige Oligonukleotide haben typischerweise eine Länge von mindestens 15 Nukleotiden.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Anleitungen zur Amplifikation von DNA-Sequenzen mit Hilfe der Polymerase-Kettenreaktion (PCR) findet der Fachmann unter anderem im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994).

Die Erfinder fanden heraus, daß coryneforme Bakterien nach Überexpression des glk-Gens in verbesserter Weise L-Aminosäuren, insbesondere L-Lysin, produzieren.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Aminosäure-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Beispielhaft wurde das erfindungsgemäße glk-Gen mit Hilfe von Plasmiden überexprimiert.

Als Plasmide eignen sich solche, die in coryneformen Bakterien repliziert werden. Zahlreiche bekannte Plasmidvektoren wie z. B. pZl (Menkel et al., Applied and Environmental Microbiology (1989) 64: 549-554), pEKExl (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z. B. solche, die auf pCG4 (US-A 4,489,160) oder pNG2 (Serwold-Davis et al., FEMS Microbiology Letters 66, 119-124 (1990)) oder pAG1 (US-A 5,158,891) beruhen, können in gleicher Weise verwendet werden.

Weiterhin eignen sich solche Plasmidvektoren mit Hilfe derer man das Verfahren der Genamplifikation durch Integration in das Chromosom anwenden kann, so wie es beispielsweise von Reinscheid et al. (Applied and Environmental Microbiology 60, 126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen bespielsweise pSUP301 (Simon et al., Bio/Technology 1, 784-791 (1983)), pKl8mob oder pK19mob (Schäfer et al., Gene 145, 69-73 (1994)), pGEM-T (Promega corporation, Madison, WI, USA), pCR2.1-TOPO (Shuman (1994). Journal of Biological Chemistry 269:32678-84; US-Patent 5,487,993), pCR®Blunt (Firma Invitrogen, Groningen, Niederlande; Bernard et al., Journal of Molecular Biology, 234: 534-541 (1993)) oder pEMl (Schrumpf et al, 1991, Journal of Bacteriology 173:4510-4516) in Frage. Der Plasmidvektor, der das zu amplifizierende Gen enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiological Letters 123, 343-347 (1994)) beschrieben. Nach homologer Rekombination mittels eines "cross over"-Ereignisses enthält der resultierende Stamm mindestens zwei Kopien des betreffenden Gens.

Ein weiterer Gegenstand der Erfindung ist demnach ein Verfahren zur fermentativen Herstellung von L-Aminosäuren, insbesondere L-Lysin, bei dem man einen mit einem Plasmidvektor transformierten Stamm einsetzt, und der Plasmidvektor die Nukleotidsequenz des für das Enzym Glukokinase codierenden Gens trägt.

Zusätzlich kann es für die Produktion von L-Aminosäuren, insbesondere L-Lysin, vorteilhaft sein, neben dem glk-Gen weitere Gene des Biosyntheseweges der gewünschten L-Aminosäure zu verstärken, damit eines oder mehrere Enzyme des jeweiligen Biosyntheseweges, der Glykolyse, der Anaplerotik, des Zitronensäure-Zyklus oder des Aminosäure-Exports überexprimiert wird.

So kann beispielsweise für die Herstellung von L-Lysin
- gleichzeitig das für die Dihydrodipicolinat-Synthase codierende dapA-Gen (EP-B 0 197 335), oder
- gleichzeitig ein für eine feed back resistente Aspartatkinase codierendes lysC-Gen (Kalinowski et al. (1990), Molecular and General Genetics 224: 317-324), oder
- gleichzeitig das für die Glyceraldehyd-3-Phosphat-Dehydrogenase codierende gap-Gen (Eikmanns (1992), Journal of Bacteriology 174:6076-6086), oder
- gleichzeitig das für die Triosephosphat-Isomerase codierende tpi-Gen(Eikmanns (1992), Journal of Bacteriology 174:6076-6086), oder
- gleichzeitig das für die 3-Phosphatglycerat-Kinase codierende pgk-Gen(Eikmanns (1992), Journal of Bacteriology 174:6076-6086), oder
- gleichzeitig das für die Pyruvat-Carboxylase codierende pyc-Gen(Eikmanns (1992), Journal of Bacteriology 174:6076-6086), oder
- gleichzeitig das für die Malat-Chinon-Oxidoreduktase codierende mqo-Gen (Molenaar et al., European Journal of Biochemistry 254, 395 - 403 (1998)), oder
- gleichzeitig das für den Lysin-Export codierende lysE-Gen (DE-A-195 48 222)
überexprimiert werden.

Weiterhin kann es für die Produktion von L-Aminosäuren, insbesondere L-Lysin, vorteilhaft sein, neben dem glk-Gen gleichzeitig
- das für die Phosphoenolpyruvat-Carboxykinase codierende pck-Gen (DE 199 50 409.1; DSM 13047) und/oder
- das für die Glucose-6-Phosphat-Isomerase codierende pgi-Gen (US 09/396,478; DSM 12969)
abzuschwächen.

Weiterhin kann es für die Produktion von L-Aminosäuren, insbesondere L-Lysin, vorteilhaft sein, neben der Überexpression des glk-Gens unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren, insbesondere L-Lysin, kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z. B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden natriumhaltigen Salze verwendet werden. Das Kulturmedium muß weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten werden Sauerstoff oder Sauerstoff haltige Gasmischungen wie z. B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum an Lysin gebildet hat. Dieses Ziel wird normalerweise innerhalb von 10 Stunden bis 160 Stunden erreicht.

Ein weiterer Gegenstand der Erfindung ist demnach ein Verfahren zur fermentativen Herstellung von L-Aminosäuren, insbesondere L-Lysin, bei dem man folgende Schritte durchführt:
a) Fermentation von die L-Aminosäure produzierenden coryneformen Bakterien, in denen man zumindest das für das Enzym Glukokinase codierende Gen verstärkt, insbesondere überexprimiert.
b) Anreicherung der L-Aminosäure im Medium oder in den Zellen der Bakterien und
c) Isolieren der L-Aminosäure.

Die Analyse von L-Lysin kann durch Anionenaustauschchromatographie mit anschließender Ninhydrin Derivatisierung erfolgen, so wie bei Spackman et al. (Analytical Chemistry, 30, (1958), 1190) beschrieben.

Das erfindungsgemäße Verfahren dient zur fermentativen Herstellung von L-Aminosäuren, insbesondere L-Lysin.

### Beispiele

Die vorliegende Erfindung wird im folgenden anhand von Ausführungsbeispielen näher erläutert.

### Beispiel 1

### Herstellung einer genomischen Cosmid-Genbank aus Corynebacterium glutamicum ATCC 13032

Chromosomale DNA aus Corynebacterium glutamicum ATCC 13032 wurde wie bei Tauch et al. (1995, Plasmid 33:168-179) beschrieben isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Molecular Biochemicals, Mannheim, Deutschland, Produktbeschreibung SAP, Code no. 1758250) dephosphoryliert. Die DNA des Cosmid-Vektors SuperCos1 (Wahl et al. (1987) Proceedings of the National Academy of Sciences USA 84:2160-2164), bezogen von der Firma Stratagene (La Jolla, USA, Produktbeschreibung SuperCosl Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert. Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt. Zur Infektion des E. coli Stammes NM554 (Raleigh et al. 1988, Nucleic Acid Research 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A Laboratory Manual, Cold Spring Harbor) beschrieben durchgeführt, wobei die Zellen auf LB-Agar (Lennox, 1955, Virology, 1:190) mit 100 µg/ml Ampicillin ausplattiert wurden. Nach Inkubation über Nacht bei 37°C wurden rekombinante Einzelklone selektioniert.

### Beispiel 2

### Isolierung und Sequenzierung des glk-Gens

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Molecular Biochemicals, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Germany). Die DNA des Sequenziervektors pZero-1 bezogen von der Firma Invitrogen (Groningen, Niederlande, Produktbeschreibung Zero Background Cloning Kit, Product No. K2500-01) wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5aMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 µg/ml Zeocin ausplattiert. Die Plasmidpräparation der rekombinanten Klone erfolgte mit dem Biorobot 9600 (Product No. 900200, Qiagen, Hilden, Deutschland). Die Sequenzierung erfolgte nach der Dideoxy-Kettenabbruch-Methode von Sanger et al. (1977, Proceedings of the National Academy of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems(Product No. 403044, Weiterstadt, Deutschland) verwendet. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Deutschland).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZerol-Derivate wurden zu einem zusammenhängenden Contig assembliert. Die computergestützte Codierbereichsanalyse wurden mit dem Programm XNIP (Staden, 1986, Nucleic Acids Research, 14:217-231) angefertigt. Weitere Analysen wurden mit den "BLAST search programs" (Altschul et al., 1997, Nucleic Acids Research, 25:3389-3402), gegen die non-redundant Datenbank des "National Center for Biotechnology Information" (NCBI, Bethesda, MD, USA) durchgeführt.

Die erhaltene Nukleotidsequenz ist in SEQ ID No. 1 dargestellt. Die Analyse der Nukleotidsequenz ergab ein offenes Leseraster von 969 Basenpaaren, welches als glk-Gen bezeichnet wurde. Das glk-Gen codiert für ein Protein von 323 Aminosäuren.

### Beispiel 3

### Herstellung eines Shuttlevektors pEC-K18mob2glkexp zur Verstärkung des glk-Gens in C. glutamicum

### 3.1. Klonierung des glk-Gens

Aus dem Stamm ATCC 13032 wurde nach der Methode von Eikmanns et al. (Microbiology 140: 1817-1828 (1994)) chromosomale DNA isoliert. Aufgrund der aus Beispiel 2 für C. glutamicum bekannten Sequenz des glk-Gens wurden die folgenden Oligonukleotide für die Polymerase Kettenreaktion ausgewählt:
glk-ex1: 5` ACT GAC GTG AGC CAG AAC 3` glk-ex2:

5` GAT CTA TCT AGA CAC CTA GTT GGC TTC CAC 3`

Die dargestellten Primer wurden von der Firma ARK Scientific GmbH Biosystems (Darmstadt, Deutschland) synthetisiert und nach der Standard-PCR-Methode von Innis et al. (PCR protocols. A guide to methods and applications, 1990, Academic Press) mit Pwo-Polymerase der Firma Roche Diagnostics GmbH (Mannheim, Deutschland) die PCR Reaktion durchgeführt. Mit Hilfe der Polymerase-Kettenreaktion ermöglichen die Primer die Amplifikation eines ca. 1,45 kb großen DNA-Fragmentes, welches das glk-Gen mit der potentiellen Promotor-Region trägt. Die DNA Sequenz des amplifizierten DNA Fragmentes wurde durch Sequenzierung überprüft.

### 3.2. Herstellung des E. coli - C. glutamicum Shuttle Vektors pEC-K18mob2

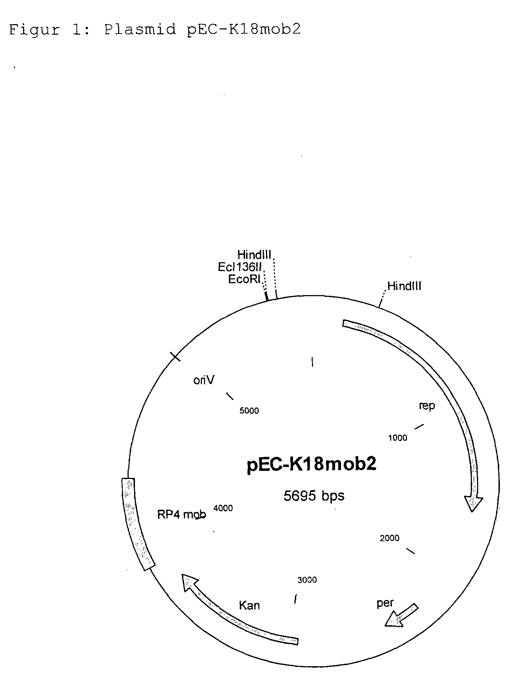

Nach dem Stand der Technik wurde der E. coli - C. glutamicum Shuttle-Vektor konstruiert. Der Vektor enthält die Replikationsregion rep des Plasmids pGA1 einschließlich des Replikationseffectors per (US-A- 5,175,108; Nesvera et al., Journal of Bacteriology 179, 1525-1532 (1997)), das Kanamycinresistenz vermittelnde aph(3')-IIa-Gen des Transposons Tn5 (Beck et al., Gene 19, 327-336 (1982)), die Replikationsregion oriV des Plasmids pMB1 (Sutcliffe, Cold Spring Harbor Symposium on Quantitative Biology 43, 77-90 (1979)), das lacZα Genfragment einschließlich des lac-Promotors und einer Mehrfachklonierschnittstelle (multiple cloning site, mcs) (Norrander, J.M. et al., Gene 26, 101-106 (1983)) und die mob-Region des Plasmids RP4 (Simon et al., Bio/Technology 1:784-791 (1983)). Der konstruierte Vektor wurde in den E. coli Stamm DH5α (Hanahan, In: DNA Cloning. A Practical Approach. Vol. I, IRL-Press, Oxford, Washington DC, USA) transformiert. Die Selektion von Plasmid-tragenden Zellen erfolgte durch Ausplattieren des Transformationsansatzes auf LB Agar (Sambrook et al., Molecular cloning: a laboratory manual. 2^{nd} Ed. Cold Spring Harbor Laboratory Press, Cold Spring Harbor, N.Y.), der mit 25 mg/l Kanamycin supplementiert worden war. Plasmid-DNA wurde aus einer Transformante mit Hilfe des QIAprep Spin Miniprep Kit der Firma Qiagen isoliert und durch Restriktion mit dem Restriktionsenzym EcoRI und HindIII mit anschließender Agarosegel-Elektrophorese (0,8%) überprüft. Das Plasmid wurde pEC-K18mob2 genannt und ist in Figur 1 dargestellt.

Folgender Mikroorganismus wurde bei der Deutschen Sammlung für Mikrorganismen und Zellkulturen (DSMZ, Braunschweig, Deutschland) gemäß Budapester Vertrag hinterlegt:
- C. glutamicum Stamm DSM 5715/pEC-K18mob2 als DSM 13245

### 3.3. Klonierung von glk in den E. coli-C. glutamicum Shuttle Vektor pEC-K18mob2

Als Vektor wurde der in Beispiel 3.2 beschriebene E. coli - C. glutamicum Shuttle-Vektor pEC-K18mob2 verwendet. DNA dieses Plasmids wurde mit dem Restriktionsenzym Ecl136II vollständig gespalten und anschließend mit shrimp alkalischer Phosphatase (Roche Diagnostics GmbH, Mannheim, Deutschland, Produktbeschreibung SAP, Product No. 1758250) dephosphoryliert.

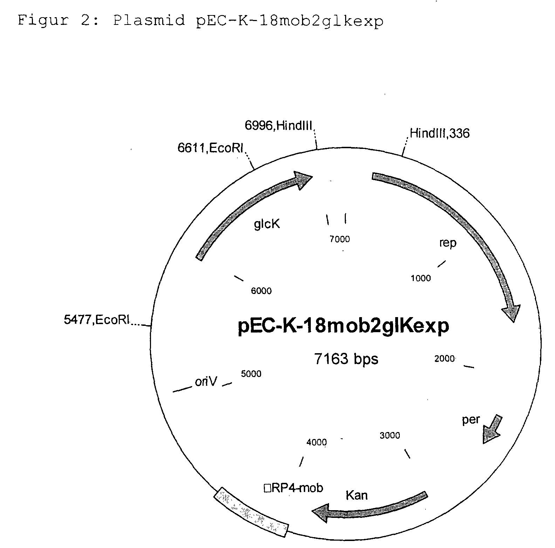

Das wie in Beispiel 3.1 beschrieben gewonnene glk-Fragment wurde mit dem vorbereiteten Vektor pEC-K18mob2 gemischt und der Ansatz mit T4-DNA-Ligase (Amersham Pharmacia, Freiburg, Deutschland, Produktbeschreibung T4-DNA-Ligase, Code no.27-0870-04) behandelt. Der Ligationsansatz wurde in den E. coli Stamm DH5αmcr (Grant, 1990, Proceedings of the National Academy of Sciences U.S.A., 87:4645-4649) transformiert. Die Selektion von Plasmid-tragenden Zellen erfolgte durch Ausplattieren des Transformationsansatzes auf LB-Agar (Lennox, 1955, Virology, 1:190) mit 25 mg/l Kanamycin. Nach Inkubation über Nacht bei 37°C wurden rekombinante Einzelklone selektioniert. Plasmid DNA wurde aus einer Transformante mit dem Qiaprep Spin Miniprep Kit (Product No. 27106, Qiagen, Hilden, Germany) nach Herstellerangaben isoliert und mit den Restriktionsenzymen EcoRI und XbaI gespalten, um das Plasmid durch anschließende Agarosegel-Elektrophorese zu überprüfen. Das erhaltene Plasmid wurde pEC-K18mob2glkexp genannt. Es ist in Figur 2 dargestellt.

### Beispiel 4

### Transformation des Stammes Corynebacterium glutamicum RES167 mit dem Plasmid pEC-K18mob2glkexp.

Der Stamm C. glutamicum RES167 (Schäfer, A. et al., Journal Bacteriological 176: 7309-731(1994)) wurde mit dem Plasmid pEC-K18mob2glkexp unter Anwendung der von Liebl et al., (FEMS Microbiology Letters, 53:299-303 (1989)) beschriebenen Elektroporationsmethode transformiert. Die Selektion der Transformanten erfolgte auf LBHIS Agar bestehend aus 18,5 g/l Brain-Heart Infusion Boullion, 0,5 M Sorbitol, 5 g/l Bacto-Trypton, 2,5 g/l Bacto-Yeast-Extract, 5 g/l NaCl und 18 g/1 Bacto-Agar, der mit 25 mg/l Kanamycin supplementiert worden war. Die Inkubation erfolgte für 2 Tage bei 33°C.

Plasmid DNA wurde aus einer Transformante nach den üblichen Methoden isoliert (Peters-Wendisch et al., , Microbiology, 144:915-927 (1998)), mit den Restriktionsendonukleasen EcoRI und XbaI geschnitten und das Plasmid durch anschließende Agarosegel-Elektrophorese überprüft. Der erhaltene Stamm wurde C.glutamicum RES167/pEC-Kl8mob2glkexp genannt.

### Beispiel 5

### Herstellung von Lysin

Der in Beispiel 4 erhaltene Stamm C. glutamicum RES167/pEC-Kl8mob2glkexp wurde in einem zur Produktion von Lysin geeigneten Nährmedium kultiviert und der Lysingehalt im Kulturüberstand bestimmt.

Dazu wurde der Stamm zunächst auf Agarplatte mit dem entsprechenden Antibiotikum (Hirn-Herz-Agar mit Kanamycin (25 mg/l)) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII verwendet.

| Medium Cg III | |
|---|---|
| NaCl | 2,5 g/l |
| Bacto-Pepton | 10 g/l |
| Bacto-Yeast-Extrakt | 10 g/l |
| Glucose (getrennt autoklaviert) | 2% (w/v) |

Der pH-Wert wurde auf pH 7.4
eingestellt

Diesem wurde Kanamycin (25 mg/l) zugesetzt. Die Vorkultur wurde 16 Stunden bei 33°C bei 240 rpm auf dem Schüttler inkubiert. Von dieser Vorkultur wurde eine Hauptkultur angeimpft, so daß die Anfangs-OD (660nm) der Hauptkultur 0,05 betrug. Für die Hauptkultur wurde das Medium MM verwendet.

| Medium MM | |
|---|---|
| CSL (Corn Steep Liquor) | 5 g/l |
| MOPS (Morpholinopropansulfonsäure) | 20 g/l |
| Glucose (getrennt autoklaviert) | 50 g/l |
| (NH₄)₂SO₄ | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0 mg/l |
| Biotin (sterilfiltriert) | 0,3 mg/l |
| Thiamin * HCl (sterilfiltriert) | 0,2 mg/l |
| CaCO₃ | 25 g/l |

CSL, MOPS und die Salzlösung wurden mit Ammoniakwasser auf pH 7 eingestellt und autoklaviert. Anschließend wurden die sterilen Substrat- und Vitaminlösungen zugesetzt, sowie das trocken autoklavierte CaCO₃.

Die Kultivierung erfolgt in 10 ml Volumen in einem 100 ml Erlenmeyerkolben mit Schikanen. Es wurde Kanamycin (25 mg/l) zugesetzt. Die Kultivierung erfolgte bei 33°C und 80% Luftfeuchte.

Nach 72 Stunden wurde die OD bei einer Meßwellenlänge von 660 nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuchs dargestellt.

**Tabelle 1**

| Stamm | OD(660) | Lysin-HCl mg/l |
|---|---|---|
| C.glutamicum RES167 | 13,8 | 287 |
| C.glutamicum RES167 /pEC-K18mob2glkexp | 15 | 345 |

Folgende Figuren sind beigefügt:
Figur 1: Karte des Plasmids pEC-K18mob2
Figur 2: Karte des Plasmids pEC-K18mob2glkexp

Die verwendeten Abkürzungen und Bezeichnungen haben folgende Bedeutung:
- per:: Gen zur Kontrolle der Kopienzahl aus pGA1
- oriV:: ColE1-ähnlicher Origin aus pMB1
- rep:: Plasmidkodierte Replikationsregion aus C. glutamicum Plasmid pGA1
- RP4mob:: RP4-Mobilisierungs-Site
- Kan:: Resistenzgen für Kanamycin
- glk:: glk-Gen von C.glutamicum

- EcoRI:: Schnittstelle des Restriktionsenzyms EcoRI
- HindIII:: Schnittstelle des Restriktionsenzyms HindIII
- Ecl136II: Ecl136II: Schnittstelle des Restriktionsenzyms

## Patentansprüche

1. Isoliertes Polynukleotid aus coryneformen Bakterien, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70 % identisch ist mit der Aminosäuresequenz von SEQ ID No.2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c).

2. Polynukleotid gemäß Anspruch 1, wobei das Polynukleotid eine in coryneformen Bakterien replizierbare, bevorzugt rekombinante DNA ist.

3. Polynukleotid gemäß Anspruch 1, wobei das Polynukleotid eine RNA ist.

4. Replizierbare DNA gemäß Anspruch 2, enthaltend
(i) die Nukleotidsequenz, gezeigt in SEQ ID No. 1, oder
(ii) mindestens eine Sequenz, die der Sequenz (i) innerhalb des Bereichs der Degeneration des genetischen Codes entspricht, oder
(iii) mindestens eine Sequenz, die mit der zur Sequenz (i) oder (ii) komplementären Sequenz hybridisiert, und gegebenenfalls
(iv) funktionsneutrale Sinnmutationen in (i).

5. Polynukleotidsequenz gemäß Anspruch 2, die für ein Polypeptid codiert, das die Aminosäuresequenz in SEQ ID No. 2 darstellt, enthält.

6. Vektor, enthaltend eine Polynukleotidsequenz nach Anspruch 1.

7. Coryneformes Bakterium, enthaltend einen Vektor nach Anspruch 6.

8. Verfahren zur fermentativen Herstellung von L-Aminosäuren, **dadurch gekennzeichnet,** daß man folgende Schritte durchführt:
a) Fermentation der die L-Aminosäure produzierenden coryneformen Bakterien, in denen man zumindest das für das Enzym Glukokinase codierende Gen verstärkt, insbesondere überexprimiert.
b) Anreicherung der L-Aminosäure im Medium oder in den Zellen der Bakterien und
c) Isolieren der L-Aminosäure.

9. Verfahren gemäß Anspruch 8, **dadurch gekennzeichnet,** daß man Bakterien einsetzt, in denen man zusätzlich weitere Gene des Biosyntheseweges der gewünschten L-Aminosäure verstärkt.

10. Verfahren gemäß Anspruch 8, **dadurch gekennzeichnet,** daß man Bakterien einsetzt, in denen die Stoffwechselwege zumindest teilweise ausgeschaltet sind, die die Bildung der L-Aminosäure verringern.

11. Verfahren gemäß Anspruch 8, **dadurch gekennzeichnet,** daß man einen mit einem Plasmidvektor transformierten Stamm einsetzt, und der Plasmidvektor die Nukleotidsequenz des für das Enzym Glukokinase codierenden Gens trägt.

12. Verfahren gemäß einem oder mehreren der Ansprüche 8 bis 11, **dadurch gekennzeichnet,** daß man coryneforme Bakterien verwendet, die L-Lysin herstellen.

13. Verfahren gemäß Anspruch 9, **dadurch gekennzeichnet,** daß gleichzeitig das für die Dihydrodipicolinat-Synthase codierende dapA-Gen überexprimiert wird.

14. Verfahren gemäß Anspruch 9, **dadurch gekennzeichnet,** daß gleichzeitig ein für eine feed back resistente Aspartatkinase codierendes lysC-Gen überexprimiert wird.

15. Verfahren gemäß Anspruch 9, **dadurch gekennzeichnet,** daß gleichzeitig das für die Gyceraldehyd-3-Phosphat-Dehydrogenase codierende gap-Gen überexprimiert wird.

16. Verfahren gemäß Anspruch 9, **dadurch gekennzeichnet,** daß gleichzeitig das für die Triosephosphat-Isomerase codierende tpi-Gen überexprimiert wird.

17. Verfahren gemäß Anspruch 9, **dadurch gekennzeichnet,** daß gleichzeitig das für die 3-Phosphatglycerat-Kinase codierende pgk-Gen überexprimiert wird.

18. Verfahren gemäß Anspruch 9, **dadurch gekennzeichnet,** daß gleichzeitig das für die Pyruvat-Carboxylase codierende pyc-Gen überexprimiert wird.

19. Verfahren gemäß Anspruch 9, **dadurch gekennzeichnet,** daß gleichzeitig das das für die Malat-Chinon-Oxidoreduktase codierende mqo-Gen überexprimiert wird.

20. Verfahren gemäß Anspruch 9, **dadurch gekennzeichnet,** daß gleichzeitig das für den Lysin-Export codierende lysE-Gen überexprimiert wird.

21. Verfahren gemäß Anspruch 10, **dadurch gekennzeichnet,** daß gleichzeitig das für die Glucose-6-Phosphat-Isomerase codierende pgi-Gen abgeschwächt wird.

22. Verfahren gemäß Anspruch 10, **dadurch gekennzeichnet,** daß gleichzeitig das für die Phosphoenolpyruvat-Carboxykinase codierende pck-Gen abgeschwächt wird.
